# EUROPÄISCHE PATENTSCHRIFT

(11) **EP 2 629 660 B1**
(45) Veröffentlichungstag und Bekanntmachung des Hinweises auf die Patenterteilung: **15.07.2020**
(21) Anmeldenummer: 11776080.1
(22) Anmeldetag: 04.08.2011
(51) Int. Cl.: A61B 5/03

(54) **IMPLANTAT ZUR MESSUNG DES INTRAKORPORALEN DRUCKES MIT TELEMETRISCHER MESSWERTÜBERTRAGUNG**
IMPLANT FOR MEASURING THE INTRACORPOREAL PRESSURE, FEATURING TELEMETRIC TRANSMISSION OF MEASURED VALUES
IMPLANT DESTINÉ À MESURER LA PRESSION INTRACORPORELLE ET PRÉSENTANT UNE TRANSMISSION TÉLÉMÉTRIQUE DES VALEURS DE MESURE

(30) Priorität: 22.10.2010 DE 102010049150
(43) Veröffentlichungstag der Anmeldung: 28.08.2013
(73) Patentinhaber: C.Miethke GmbH&Co Kg, 1469 Potsdam (DE)
(72) Erfinder: MIETHKE, Christoph, 14469 Potsdam (DE)
(74) Vertreter: Greiche, Albert
(86) Internationale Anmeldenummer: PCT/EP2011/003903
(87) Internationale Veröffentlichungsnummer: WO 2012/052078

(56) Entgegenhaltungen:
- WO-A-2006/117123
- DE-A1-102007 024 270
- DE-A1-102007 056 844
- DE-A1-102008 033 337
- DE-A1-102008 037 736
- DE-B3-102007 008 642

## Beschreibung

Im Rahmen neurochirurgischer Interventionen kommt der Erfassung des intracraniellen Druckes eine herausragende Bedeutung zu.

Zahlreiche Erfindungen haben sich die Lösung dieses Problems zur Aufgabe gemacht; alle bisher erhältlichen Systeme weisen aber gravierende Nachteile auf, die zu überwinden sich die hier vorgestellte Erfindung zur Aufgabe gemacht hat.

Durchgesetzt in der klinischen Praxis haben sich invasive Meßmethoden, bei denen ein Sensor in den Körper eingebracht wird, wobei über eine Kabelverbindung das Signal an ein externes Gerät zur Darstellung und Auswertung des Messwertes weitergeleitet wird. Häufig geschieht dies im Zusammenspiel mit einer künstlichen Drainageleitung, durch die Hirnwasser extrakorporal abgeleitet werden soll. Kritisch bei solchen Systemen ist die sehr hohe Infektionsgefahr. Lange Verläufe können nur durch sehr aufwendige Infektionsprophylaxe und den mehrfachen Austausch des Drucksensors realisiert werden. Insbesondere bei Patienten mit Hydrocephalus ist aber gerade der klinikferne Verlauf des Hirndruckes nach Implantation eines internen künstlichen Drainagesystems von großem diagnostischem Interesse. Für solche Aufgaben eignen sich Systeme, die das gemessene Signal durch die intakte Haut weiterleiten oder aber die Messung durch die Haut ermöglichen.

In Patent DE 196 38 813 C1 wird ein implantierbarer Drucksensor beschrieben, der mit flexiblen Folienleiterbahnen verbunden ist und von einem Substrat im Bereich des Sensorelementes umgeben ist, das eine höhere mechanische Festigkeit aufweist als die Folienleiterbahn und das zusammen mit dem Sensorelement in eine flexible Masse eingeschlossen ist. Durch den Aufbau soll eine zuverlässige und kostengünstige Messvorrichtung möglich werden, die jedoch wegen der erforderlichen Hautdurchleitung hinsichtlich des Infektionsrisikos keine Minderung bietet. Im Zusammenhang mit der Sensorik wird Bezug genommen auf Patent US 4,738,267, in dem eine Kunststoffkapsel mit Membran verwendet wird, auf die ein Dehnungsmessstreifen aufgebracht wird. Die Verstimmung der Wheatstone-Brücke wird als das Maß für den anliegenden Druck interpretiert. Ein solcher Sensor arbeitet ungenau und weist ein unakzeptabel hohes Driftverhalten auf. Aus diesem Grunde hat er sich als implantierbarer Hirndrucksensor nicht durchgesetzt.

Als Zusatzanmeldung zu DE 196 38 813 C1 wird in der Anmeldung DE 197 05 474 die analoge Technik auch zur telemetrischen Erfassung von intracorporalen Drücken beschrieben. Allerdings sind hier keine Hinweise gegeben, wie die Biokompatibilität sichergestellt werden soll. Ein solcher Sensor ist bis heute nicht zur Marktreife gelangt.

Ein gleichfalls telemetrisches Verfahren zur Erfassung des intracraniellen Druckes wird in der Patentschrift US 611 3553 beschrieben. Die hier angemeldeten Ansprüche beziehen sich auf die möglichst driftfreie und langzeitstabile Messung unter Beschreibung des elektronischen Aufbaus. Verwendung findet hier ein kapazitiver Aufbau, wobei der Sensor im Knochen des Patienten versenkt werden soll. Dies ist erforderlich aufgrund des raumgreifenden Aufbaus des Sensors. Inwieweit die tatsächlichen Eigenschaften des Sensorkonzeptes den hohen Anforderungen an die Hirndruckmessung hinsichtlich Genauigkeit und Driftverhalten genügen ist nicht bekannt, da ein solcher Sensor bisher nicht marktverfügbar ist und somit nicht unabhängigen Tests unterzogen werden konnte.

Ein Verfahren zur Erfassung des intracraniellen Druckes ohne Hautdurchleitung ist ebenfalls in Patent US 4,676,255 aus dem Jahre 1987 beschrieben. Ein unter der Haut platzierter Sensor hat seine Nullposition solange intracraniell gegenüber der Umgebung kein positiver oder negativer Differenzdruck anliegt. Steigt oder fällt der intracranielle Druck bewegt sich der Sensor aus der Nullposition. Von außen wird nun durch die Haut genau der Druck aufgebracht, der nötig ist, um den Sensor wieder in die Nullposition zu bringen. Der dazu notwendige Druck soll dann dem intracraniellen Druck entsprechen. Dieses Verfahren hat sich klinisch nicht durchsetzen können. Gründe hierfür sind die Varianz der Haut von Patient zu Patient, die technisch schwierige und fehlerhafte Erfassung der Nullposition sowie die komplizierte Erzeugung des notwendigen externen Druckkissens.

Im Patent DE 198 58 172 wird eine Methode beschrieben, die auf mikrosystemtechnischer Basis den intracorporalen Druck unmittelbar mittels eines Sensorelementes erfasst. Im Mittelpunkt der Betrachtungen steht die Erfassung des Augeninnendruckes. Das Implantat soll daher möglichst klein und leicht sein. Bei der Verwendung dieser Technologie zur Erfassung des intracraniellen Druckes kommt der Beschichtung des Sensors eine entscheidende Bedeutung zu. In Patentschrift DE101 56 494 wird ein solcher Sensor beschrieben, bei dem zur Gewährleistung der Biokompatibilität eine Metallschicht sowie zumindest abschnittsweise eine biokompatible Kunststoffschicht vorgesehen ist. Ein solcher Aufbau weist erhebliche Nachteile auf. Eine solche Beschichtung des Sensorelementes ermöglicht die Beeinträchtigung der Messung wegen der Durchleitung durch eben diese Schicht, deren Eigenschaften sich im Laufe der Zeit verändern können. Die Schicht kann beschädigt werden aufgrund von Krafteinwirkungen von außen. Zumindest ist das Driftverhalten aufgrund von Alterung insbesondere der Kunststoffschichten problematisch.

Im Patent EP 1312 302 A2 wird eine Technik beschrieben, bei dem ein um den Sensor angeordnetes Medium von einer flexiblen Hülle umgeben ist. Wie die Biokompatibilität der flexiblen Hülle gewährleistet werden soll, ist in der Schrift nicht beschrieben. Die in der Anmeldung favorisierte Verwendung von Silikonöl zur optimalen Übertragung des anliegenden Druckes erscheint unter Berücksichtigung von Risikoaspekten problematisch.

Ein anderes, in der EP1312302 beschriebenes Gerät setzt sich aus folgenden Merkmalen zusammen:
a) intrakorporale Hirndruckmessung
b) mit implantierbarer Meßeinrichtung
c) mit einem Sensorelement
d) mit einer Telemetrieeinheit, bestehend aus einer induktiven Spule
e) mit einem Träger, in oder auf dem das Sensorelement und die Telemetrieeinheit angeordnet sind
e) mit einer flexiblen Umhüllung von Träger, Telemetrieeinheit und Sensorelement
f) mit einem anschließbaren extrakorporalen Auswerteeinrichtung und
g) einem anschließbaren telemetrischen Abfrage/Lesegerät Dabei ist darauf zu verweisen, dass die EP1312302 im Wesentlichen auf die Verwendung einer Flüssigkeit als Druckübertragungsmittel zielt, obwohl in EP1312302 auch die Möglichkeit der Verwendung von Gas als Druckübertragungsmittel angesprochen ist. Die Flüssigkeit als Druckübertragungsmittel hat für den Fachmann den Reiz einer praktischen Unkomprimierbarkeit. Druckänderungen werden dadurch sofort und ohne Änderungen weitergegeben. Die Präferenz für flüssige Druckmittel ist in EP1312302 unverkennbar.

In der US6673022B1 ist gleichfalls eine implantierbare Vorrichtung zur Hirndruckmessung bekannt. US6673022 spricht von einer Blase aus weichem Material am Ende einer katheterförmigen Vorrichtung. Das weiche Materiall verformt sich im Betriebszustand unkontrolliert, so daß die Zuverlässigkeit der Messung fraglich ist.

Die Messeinrichtung sitzt bei der bekannten Vorrichtung an dem der Spitze gegenüberliegenden Ende der Vorrichtung. Die Blase schließt ein Luftvolumen ein..

Auch die DE102005020569 geht von einer implantierbaren Messeinrichtung aus, die ein Sensor enthält, wobei die Messeinrichtung mit einer flexiblen Umhüllung versehen ist und aus einem Druckaufnehmer besteht und wobei der Druckaufnehmer (wahlweise gemeinsam mit der Telemetrieelektronik) in einem Chip angeordnet ist und in einem Druckmedium eingebettet sind, wobei die Umhüllung das Druckmedium umschließt. Die hier verwendete Bezeichnung "einbetten/umhüllen" umfasst auch eine nur teilflächige Belastung/Beaufschlagung des Druckaufnehmers mit dem Druckmedium.

Als Stand der Technik ist auch die 102005020569 zu berücksichtigen, welche die Grundlage für die WO2006/11712 bildet. Soweit im Folgenden die DE102005020569 angesprochen ist, schließt das die WO2006/11712 ein.

Die DE102005020569 beschreibt eine kleinvolumige Umhüllungen einer implantierbaren Messeinrichtung, wobei die Messeinrichtung in Flüssigkeit oder Gas eingebettet ist, vorzugsweise mit telemetrischer Messwertübertragung, noch weiter bevorzugt mit metallischen Gehäusen und einem Fenster für eine Druckübertragung auf die Messeinrichtung, wobei das Fenster mit einer Membran verschlossen ist, insbesondere mit einer metallischen Membran. Dabei soll ein miniaturisierter Chip zur Erfassung des Absolutdruckes so in ein Gehäuse platziert werden, dass einerseits die Biokompatibilität des Implantats auch langfristig sichergestellt ist und dass andererseits eine möglichst driftfreie und hochgenaue Messung erfolgen kann. Die DE102005020569 soll dabei die Schwierigkeiten bei der Passivierung des elektronisch arbeitenden Sensors überwinden, die insbesondere in der Sicherheit des Schutzes im Hinblick auf Alterung oder Beschädigung, in der Beeinträchtigung der Druckübertragung durch die aufgebrachte Schutzschicht und in der sich aus im Laufe der Zeit einstellenden Materialänderungen ergebenden unkalkulierbaren Drift gesehen werden.

Um diese Probleme zu überwinden wird in der DE102005020569 vorgeschlagen, den Sensor in ein metallisches Gehäuse, insbesondere aus Titan einzubringen. Über eine oberhalb des Sensors angebrachte dichte, biokompatible möglichst elastische Membrane wird der außen am Gehäuse anliegende Druck auf das Innere des Sensors übertragen. Vorzugsweise besteht die Membran gleichfalls aus Titan. Aber auch andere Membranen kommen in Betracht.

Trotz der technischen Machbarkeit der Erfindung nach DE102005020569 konnte ein entsprechendes Produkt bislang nicht zur Marktreife entwickelt werden. Die Gründe hierfür liegen zum einen in wirtschaftlichen und technischen Randbedingungen, die teilweise aufwendige Fertigung führt zu hohen Herstellungskosten.

In DE10239743 sowie in diesem Zusammenhang DE102005008454 und DE102008011601 wird ein implantierbarer Sensor beschrieben, der einen miniaturisierten Sensorchip zur Messung von Druck und Temperatur an der Spitze eines Katheters trägt. Dabei sind alle elektronischen Module für die Auswertung der Sensorik, für die telemetrische Übertragung sowie die Energieversorgung auf einer Platine angebracht. Diese Platine ist gekapselt in einem Gehäuse aus implantierbaren Material untergebracht. Durch die Trennung des Sensors von der Elektronik kann der Sensor sehr klein sein. Die Trägerbaugruppe ist hier in einem Keramikgehäuse untergebracht, das unter der Kopfhaut implantiert wird, ein dünner Katheter trägt in der Spitze einen Drucksensor. Der Katheter besteht aus einem elastomeren Material. Der Sensor in einem sich verfestigenden Material vergossen. Allerdings kann ein Sensor dieser Bauart bisher nur für relativ kurze Zeit ein verwertbares Signal liefern, da die Werte u.a. durch Altern und Wasseraufnahme des Kunststoffes driften. Durch Dämpfung des Vergussmaterials wird das dynamische Verhalten negativ beeinflusst, was die Messung von Impulswellen schwierig macht. Für anliegende Unterdrücke ist die Bauweise nur sehr bedingt geeignet, da Zugbelastungen vom Vergussmaterial nicht gut übertragen werden. Das Produkt kann nur kurzzeitig, zulassungsgemäß für 28 Tage, implantiert werden, die Integration in ein bestehendes Shunt System ist nicht möglich.

Die vorliegende Erfindung solleinen Druckmesser schaffen, der ,eine statisch wie dynamisch vorteilhafte Funktion bietet, insbesondere im Hinblick auf eine sehr langfristige, driftfreie Funktion des implantierten Sensors . Vorzugsweise soll die Einrichtung integriert in ein bestehendes Shunt System zur Behandlung des Hydrocephalus einsetzbar sein.

Die Aufgabe wird mit Hilfe der Merkmale Hauptanspruches gelöst. Vorteilhafte Ausführungsformen sind Gegenstand der Unteransprüche.

Zu den Merkmalen des Hauptanspruches gehört eine gewellte, sehr spezielle Membran, wobei die Wellenabmessungen und die Membrandicke eine erhebliche Rolle spielen.

Gewellte Membranen sind an sich bekannt,
Das ergibt sich aus folgenden Druckschriften
DE102007056844
DE102007024270
DE102008037736
DE102008033337.

Die DE102007056844 betrifft einen Druckmessumformer. Der Druckmessumformer umfaßt einen Druckmittler, sowie einen Drucksensor mit einem druckempfindlichen, elastischen Verformungskörper und ein Wandlerelement zur Ausgabe eines von einer Verformung des Verformungskörper abhängigen elektrischen oder optischen Signals, wobei die Druckkammer über einen hydraulischen Pfad, welcher eine mit einer Übertragungsflüssigkeit gefüllte Leitung umfaßt, mit dem Verformungskörper des Drucksensors hydraulisch verbunden ist. Der Verformungskörper ist dabei wahlweise als gewellte Membran ausgebildet. Die DE102007056844 befaßt sich im Detail mit der Herstellung eines Membranbettes, an der die Membran unter entsprechendem Druck zur Anlage kommen kann. Das Membranbett soll eine Abbildung der Membran beinhalten, wobei die Abbildung durch Funkenerodierung erzeugt wird. Zugleich befaßt sich die DE102007056844 mit radialen Durchbrechungen der Wellen des Membranbettes.

Einen Hinweis auf die besonderen Abmessungen der Wellen bzw. der Membran und auf die Anwendung zur Messung von intracraniellen Drücken enthält die DE102007056844 nicht.

Die DE102008037736 besitzt auch eine gewellte Membran, der eine gleichermaßen gewellte Anlagefläche zugeordnet ist. Die Membran soll wie die Membran der DE102007056844 Drücke aufnehmen. Auch bei dieser Druckschrift findet sich kein Hinweis auf die besonderen Abmessungen der Wellen und auf die Anwendung zur Messung intracranieller Drücke. Hinzu kommt, daß die Membran einen Hub hat, der sich als Bewegungsantrieb für eine Steuerung oder dergleichen nutzen läßt. Die mit der erfindungsgemäßen Vorrichtung erzeugten Membranbewegungen sind dagegen nicht als Bewegungsantrieb geeignet.

Die DE102007024270 besitzt auch eine gewellte Membran, der ein gleichermaßen gewelltes Membranbett zugeordnet ist. Als Besonderheit ist in der DE102007024270 offenbart, daß der unmittelbar an die Befestigungsfläche angrenzende Randbereich der Membran gegenüber dem Membranbett beabstandet verläuft. Ein Hinweis auf die erfindungsgemäßen Abmessungen der Membran und auf deren Anwendung zur Messung intracranieller Drücke ist auch der Druckschrift nicht zu entnehmen.

Die DE102008033337 knüpft an die Offenbarung de DE102007056844 an und enthält diverse Vorschläge zur Berücksichtigung einer unsymmetrischen Membranverformung. Die unsymmetrische Membranverformung resultiert aus einer sich wölbend über einem vertieften Membranbett erstreckenden Membran und ist für die erfindungsgemäße Membran und deren Verformung irrelevant.

Es wird erfindungsgemäß eine Druckkammer verwendet, die mit einem gasförmigen Medium gefüllt ist und an einer Seite durch eine Membran vom Außenraum getrennt wird, die aus einem Metall besteht. Der gesamte Aufbau bestehend aus Druckkammer, Sensorik, Auswertung, telemetrischer Übertragung und Energieerzeugung befindet sich in einem Gehäuse. Hierdurch müssen keine Funktionsteile in den Bereich des zentralen Nervensystems geführt werden. Es sind Ventrikelkatheter geeignet, wie sie gängigerweise in Shunt Systemen zur Behandlung des Hydrocephalus verwendet werden.

Die Arbeitsweise des gasförmigen Druckmittlers lässt sich wie folgt beschreiben:
Ändert sich der Druck außerhalb des Behälters, kommt es zu einer Verformung der Membran, die bestimmt wird vom Volumen im Behälter, der Eigenschaft der Membran und dem Wert der außen wirkenden Druckänderung.

Bei einem zylinderförmigen Gehäuse, zum Beispiel einem zylinderförmigen Katheter mit am Umfang angeordneter Membran können aufgrund der durch die Wölbung entstehenden Spannungen in der Membrane die Drücke innerhalb und außerhalb des Behälters unterschiedlich sein. Allerdings gibt es dann zu jeder Membranstellung eine charakteristische Drucksituation im Behälter, die einem extern anliegenden Druck entspricht. Durch die Messung des Drucks in dem Behälter kann man also auf den äußeren Druck rückschließen. Die Absolutbewegung der gewählten Membrane ist nicht linear zu der anliegenden Druckdifferenz.

Wenn die Membran stirnseitig an einem zylinderförmigen Gehäuse angeordnet ist, gibt es bei ebener Strirnfläche eine mögliche Membranstellung, in der die Membran gleichfalls eine ebene Fläche bildet. In allen anderen Membranstellungen besitzt die Membran eine Wölbung(Auswölbung oder Einwölbung), für die vergleichbare Regeln wie zu einer am Umfang eines zylinderförmigen Gehäuses angeordneten Membran gelten.

Nach der Erfindung wird eine besondere Flexibilität der Membranfläche durch eine Wellenform der Membranfläche erreicht. Es mindestens eine Welle in der Membranfläche vorgesehen. Vorzugsweise ist mindestens 1/3 der druckbeaufschlagen Membranfläche gewellt, noch weiter bevorzugt ist mindestens 2/3 der druckbeaufschlagten Membranfläche und höchst bevorzugt ist mindestens 4/5 der druckbeaufschlagten Membranfläche gewellt. Mit druckbeaufschlagter Fläche ist hier die vom äußeren Medium, dem Hirnwasser bzw. Liquor, beaufschlagte Membranfläche bezeichnet. An den Wellen kann die Membran sich verhältnismäßig leicht verformen, jedenfalls viel leichter als in einer im Ausgangszustand ebenen/flachen Folie. Damit trägt die erfindungsgemäße Membran ideal zur sicheren und driftfreien Messung von Körperdrücken, auch für lange Zeiträume, auch für miniaturisierte Microchip-Sensoren bei, die auf Siliziumbasis hergestellt werden.

Will man durch die Druckmessung in einem B'ehälter eine indirekte Druckmessung durchführen, so ist es von Vorteil, wenn die Kammer für den am häufigsten auftretenden Druck eine entspannte Membrane aufweist. Die entspannte Membran steht nicht unter Spannung. Je weniger es nun bei äußeren Druckschwankungen zu Spannungen in der Membrane kommt, desto genauer wird der Druck von außen nach innen übertragen und desto genauer wird die Messung. Im besten Falle erreicht der zu messende Druck keine Werte, bei denen es in der Membran zu wesentlichen Spannungen kommt. Je nach Eigenschaft und Form der Membrane kommt es zu keiner oder nur einer sehr geringen Spannungsänderung innerhalb der Membran. Mit der erfindungsgemäß besonders nachgiebigen Membran Lassen sich die Spannungen in der Membran minimieren.

Je kleiner die Membranfläche ist, desto größer wird der Einfluss der Membransteifigkeit auf den Messvorgang.

Die Druckkammer kann so ausgelegt sein, daß die Membran bei maximaler Auslenkung bzw. bei maximaler Verformung keine gegenüberliegende Kammerwand berührt.

Vorzugsweise ist der Abstand der Membran von der gegenüberliegenden Kammerwand aber so gewählt, daß die gegenüberliegende Kammerwand die Auswölbung der Membran begrenzt. Dann bildet die gegenüberliegende Kammerwand eine Membrananlagefläche/Berührungsfläche.

Die Membrananlagefläche kann verschiedene Formen aufweisen, eben und/oder gewölbt, trichterförmige oder hügelförmig. Im Falle einer ebenen Membrananlagefläche kommt die Membran im einer vorgesehenen Berührung zuerst mittig mit der gegenüberliegenden Kammerwand in Berührung.

Das führt bei mittig angeordneter, zu dem Druckaufnehmer führender Zuleitung im Extremfall sofort zu einem Verschluss der Zuleitung und zu einer Unterbrechung der Druckmessung. Wenn eine weitergehende Messung trotz mittiger Berührung erfolgen soll, kann dem mit verschiedenen Maßnahmen Rechnung getragen werden. Wahlweise wird die Zuleitung dazu verlegt, so dass sie mehr am Rand der Membran in den Zwisscshenraum zwischen Membran und Membrananlagefläche mündet.

Vorzugsweise ist die Membrananlagefläche so ausgestaltet, daß die erste Berührung der Membran mit der Membrananlagefläche nicht mittig sondern im Abstand von der Mitte stattfindet. Das ist zum Beispiel bei einer Trichterform der Fall. Dann verbleibt mittig ein Gasvolumen, Durch Anpassung an die Form der ausgebeulten Membran kann das Gasvolumen minimiert werden.

Alternativ oder zusätzlich kann die Membrananlagefläche mit geringen Vertiefungen versehen werden, die eine weitergehende Verbindung des Zwischenraumes zwischen Membran und Membrananlagefläche mit der zu dem Druckaufnehmer führenden Gasleitung bewirken.

Es ist zu berücksichtigen, daß die Membran je nach anliegendem Druck unterschiedlich verformt wird. Es ist von Vorteil, wenn die Membrananlagefläche einem ausgewählten Verformungszustand der Membran angepaßt ist. Die Anpassung einer gewellten Membran führt vorzugsweise zu einer gewellten Membrananlagefläche. Entsprechend der Verformung dem Membran besitzt die Membrananlagefläche eine Wellung, mit der sich die Membrananlagefläche in dem ausgewählten Verformungszustand der Membran ganz oder teilweise schließend an dem Membran anlegt und die Vertiefungen der Membran zwischen deren Wellenkämmen ausfüllt.

Der Druckaufnehmer kann in der Membrananlagefläche integriert sein. In dem Fall wird vorzugsweise ein geringer Abstand zwischen der Membran und dem Druckaufnehmer gewahrt.

Besonders bevorzugt ist der Druckaufnehmer in einer von der Druckkammer(mit der Membran) beabstandeten weiteren Kammer angeordnet, die mit dem Raum zwischen gewellter Membran und Membrananlagefläche über eine Leitung verbunden sind. Das Volumen der Druckkammer ist durch den Bauraum konstruktiv bedingt und in einen passiven und einen aktiven Raum aufteilbar. Der aktive Raum ist dabei derjenige Raum, der von der Membran bei einem anliegenden Überdruck maximal verdrängt werden kann, also der Hubraum. Im verbleibenden, unveränderlichen Teil des Kammervolumens, dem passiven Raum, befindet sich der Druckaufnehmer.

Von Vorteil ist, wenn der passive Raum größer als der aktive Hubraum ist.

Die erfindungsgemäße Druckmessung ist aber auch möglich, wenn der passive Raum größer als der aktive Hubraum ist.

Eine teilflächige Berührung der Membran an der Membrananlagefläche ist unschädlich.

Bei einer sehr steifen Membran ist es zur Erlangung einer ausreichenden Messgenauigkeit wichtig, dass der passive Teil des Kammervolumens wesentlich kleiner ist als das aktive Hubvolumen, das Verhältnis sollte idealerweise größer als 1:10 sein. Da auch insgesamt ein kleines Kammervolumen angestrebt wird, sind bei einer steifen Membran aufwendige Gestaltungen erforderlich, um das Kammervolumen in Relation zum aktiven Hubraum zu minimieren.

Bei der erfindungsgemäßen, besonders flexiblen Membran sinken die Anforderungen an dieser Stelle. Von Vorteil ist, wenn das Hubvolumen auch hier größer als der passive Raum ist. Bei Verwendung der erfindungsgemäßen, gewellten Membran kann es auch ausreichend sein, wenn der passive Raum größer als der aktive Hubraum ist. Vorzugsweise wird dabei das Verhältnis von 4:1 , noch weiter bevorzugt das Verhältnis von 2:1 nicht überschritten. Günstig ist eine Abstimmung der Membranfläche auf das Kammervolumen. Für die bevorzugte, kreisrunde Membranfläche stellen sich vorteilhafte Ergebnisse ein, wenn das Verhältnis von Höhe des Membranhubes zum Radius der Membranfläche von 1: 15 bis 1: 50, vorzugsweise 1: 25 ist. Bei anders geformten Membranen können vergleichbare Ergebnisse erwartet werden, wenn der mittlere Radius der Membranfläche zum Membranhub ins Verhältnis gesetzt wird.

Erfindungsgemäß beträgt das gesamte Kammervolumen 20 bis 350 mm³, vorzugsweise 50 bis 350 mm³. Zum Beispiel besitzt eine Bauform mit ca. 130 mm³ Kammervolumen und 40 mm³ Hubvolumen Vorteile. In dem angegebenen Kammervolumen-Größenbereich ist es von Vorteil, wenn das Membran-Hubvolumen ca. 20 - 100 mm³ beträgt.. Durch die Einführung der erfindungsgemäßen, besonders flexiblen Membran wird damit die technische Machbarkeit erleichtert, da durch das größere Kammervolumen insgesamt und insbesondere den größeren, passiven Bereich ausreichend Raum für den Einbau der erforderlichen Systemkomponenten verbleibt.

Die Form der Membran ist bevorzugt rund, es sind aber auch andere Membranformen realisierbar, von ovalen Formen bis zu mehreckigen Varianten. Besonders bevorzugt ist die Membranfläche kreisrund. Bei anderen Bauformen muss zur Ermittlung des geeigneten Größenverhältnisses Hubhöhe:Radius auf eine kreisrunde Fläche idealisiert bzw. umgerechnet werden.

Für die Membran kann eine spiralförmig oder ringförmig oder anders verlaufende Welle vorgesehen sein. Spiralförmige Wellen können eingängig oder mehrgängig sein. Vorzugsweise sind ringförmig verlaufende Wellen vorgesehen. Ringförmig verlaufende Wellen zeigen ein besonders vorteilhaftes Verformungsverhalten. Bei mehreren ringförmig verlaufenden Wellen sind die Wellen vorzugsweise mit unterschiedlichem Ringdurchmesser versehen, so dass eine konzentrische Anordnung möglich ist. Wenn sich dabei eine Welle an die andere anschließt, ist der Durchmesser an jeder Wellenmitte eines Wellenringes um das doppelte Wellenmaß größer als der eingeschlossene nächstliegende Wellenring. Wenn zwischen den Wellen noch ein Abstand vorgesehen ist, so vergrößert sich der Durchmesser an jeder Wellenmitte gegenüber dem vorstehenden Durchmesser um das doppelte Abstandsmaß.

Die Wellen können im Querschnitt unterschiedliche Formen aufweisen. Im Extremfall handelt es sich bei der Welle nur um eine Auswölbung in der einen oder anderen Richtung quer zur Membranfolienebene. Vorzugsweise ist ein sinusförmiger Wellen-Verlauf mit einer Auswölbung in der einen Richtung einer Auswölbung in der anderen Richtung quer zur Membranfolieneben vorgesehen. Das heißt, vorzugsweise verläuft die Welle wie eine sinusförmige Schwingung. Auch ein anderer Verlauf kommt in Betracht, wenn die Welle im Höchsten und im Tiefsten eine Rundung aufweist, deren Radius mindestens gleich der mehrfachen Dicke der Membranfolie ist, vorzugsweise mindestens gleich der 10fachen Dicke der Membranfolie ist, noch weiter bevorzugt mindestens gleich der 50fachen Dicke der Membranfolie, höchst bevorzugt mindestens gleich der 100fachen Dicke der Membranfolie ist.

Dabei kann die Membranfolie aus Titan eine geringe Dicke von 0,005 bis 0,05 mm vorzugsweise eine Dicke von 0,01 bis 0,03 mm besitzen.

Die gewellten Membran trägt zur Reduzierung des aktiven Hubvolumens bei, wenn die gewellte Membran sich gegen eine gewellte Fläche (Membrananlagefläche) verformen kann, so dass sich die Membran mit einer Auswölbung in eine Vertiefung der Membrananlagefläche einlegen kann und umgekehrt die Membrananlagefläche mit einer Auswölbung in Vertiefungen der Membran legen kann. Günstige Verhältnisse ergeben sich schon, wenn die Membrananlagefläche eine gleiche Wellung aufweist wie die Membran. Noch bessere Verhältnisse ergeben sich, wenn die Kontur der Membrananlagefläche der verformten Membran nachgebildet ist. Die Membran erfährt durch Druckbelastung eine Formänderung. Wenn die Membrananlagefläche dem angepasst ist, kann das Volumen zwischen Membran und Druckaufnehmer minimiert werden. Die maximale Auslenkung der Membran kann hierdurch derart begrenzt werden, dass eine Ausdehnung bis fast an die Streckgrenze des Materials erlaubt wird. Die maximale Ausdehnung ist damit abhängig von der Größe der Membran und kann bis zu 1 mm betragen, vorzugsweise liegt sie bei 0,0,05 bis 0,04 mm, noch weiter bevorzugt bei 0,01 bis 0,03mm. Dies ermöglicht einen sehr großen Messbereich für den anliegenden Druck. Bei geringen Dicken werden die Membranen aus Folien hergestellt. Bei größeren Dicken aus Blechen.

Bei den im Rahmen einer intrakraniellen Druckmessung auftretenden Drücke soll die Membran idealerweise in einem möglichst entspannten Zustand arbeiten. In einem Druckbereich von 800 bis 1200 mbar soll die Membran einer bevorzugten Bauform die Auflagefläche nicht berühren.

Da die Membran sich nach Innen verformt, gilt dies zunächst nur für Überdrücke. Funktional macht es keinen Unterschied, ob ein Unter- oder Überdruck anliegt, durch die Konstruktion sind beide Situationen mit gleicher Messgenauigkeit erfassbar. Wird die Wölbung der Membran nach außen ebenfalls durch eine der Membran angepasste, gewellte Anlagefläche begrenzt, ist auch hier eine optimale Ausformung bis an die Streckgrenze des Materials gewährleistet.

Im implantierten Zustand kommen Drücke in dieser Höhe nicht vor, es kann aber im Fertigungsprozess und hier insbesondere während der Sterilisation zu derartigen Belastungen kommen. Die Robustheit der Konstruktion erlaubt die besonders wirtschaftliche Sterilisation mittels Dampf, bei der sehr hohe Drücke bis 200 bar auftreten.

Je nach Anordnung der Membranfolie am Gehäuse kann es erforderlich sein, die Membran auch am Rand zu Verformen. Das ist zum Beispiel bei einem zylindrischen Gehäuse der Fall, welches stirnseitig von der Membran verschlossen wird. Dann kann es zweckmäßig sein, zusätzlich zur Wellform noch einen Kragen an der Membran zu erzeugen, mit dem die Membran beim Verschließen der stirnseitigen Gehäuseöffnung um den Rand der Stirnfläche herum zum Gehäusemantel zu führen.

Das Gehäuse der Messzelle ist nach allen Seiten außer der durch die Membran gebildete Fläche steif und absolut dicht. Neben der Druckkammer befinden sich im Gehäuse der Druckaufnehmer, vorzugsweise in Form eines ASIC Chips (elektronische Schaltung, bestehend aus digitalen Bauelementen und den Verbindungen zwischen diesen) sowie alle elektronischen Bauteile zur Auswertung, telemetrischen Übertragung und Energieversorgung. Die Energieversorgung erfolgt induktiv, hierzu ist im Gehäuse eine Spule angebracht. Desgleichen kann eine Batterie eingesetzt werden.

Der Druckaufnehmer ist sehr empfindlich gegenüber mechanischer Belastung.

Würde der Chip durch eine mechanische Spannung belastet sind die Messergebnisse nicht zu verwerten. Eine spannungsfreie Anordnung ist daher erwünscht. Mechanische Belastungen können durch Spannungen in der Montage, durch Bewegung oder durch thermische Ausdehnung der Komponenten entstehen. In eine Ausführung der Erfindung ist der Chip auf einer eigenen Platine montiert. Die Befestigung erfolgt vorzugsweise mittels punktueller Verklebung in der Mitte des Chips. Der Chip hat an zwei gegenüberliegenden Seiten Kontaktstellen, die zum vorzugsweise durch Bonden mit der Trägerplatine verbunden werden. Die gebondeten Verbindungen werden vorzugsweise durch einen Glop Top Verguss (thermisch härtendes Epoxidharz) geschützt. Neben dem ASIC Druckaufnehmerchip befinden sich die vorzugsweise Kondensatoren zur Spannungsregelung auf der Trägerplatine, so dass lediglich drei Leiter zur Verbindung der gesamten Platine mit der restlichen Elektronik erforderlich sind. Durch die punktuelle, mittige Verklebung des Chips und die flexible Verbindung durch Bonden ist die Belastung des Chips durch mechanische Spannungen bereits sehr gut von der Trägerplatine entkoppelt. Die Trägerplatine selber ist zusätzlich an zwei Stellen an den Längsseiten in Höhe der Kanten des ASIC Chips geschlitzt. Hierdurch wird ein Unterschied in der thermischen Ausdehnung der Platine zum Chip und dessen Anschlüssen ausgeglichen. Die Trägerplatine kann aus Keramik hergestellt sein, die einen ähnlichen Ausdehnungskoeffizient wie der Chip hat, vorzugsweise kann FR4 mit einer Stärke von 0,5 mm verwendet werden.

Auch eine zusätzliche Entkopplung der Trägerplatine ist von Vorteil. Erfindungsgemäß wird dies realisiert über eine einseitige Aufhängung der Trägerplatine auf der Hauptplatine der Messzelle. Die Aufhängung ist an der gleichen Seite angebracht, an der auch die Durchleitungen der Verbindungskabel des ASIC liegen. Diese Kabel selbst sind spiralförmig verdreht, so dass keinerlei mechanischer Widerstand entsteht und die Verbindung sehr elastisch ist.

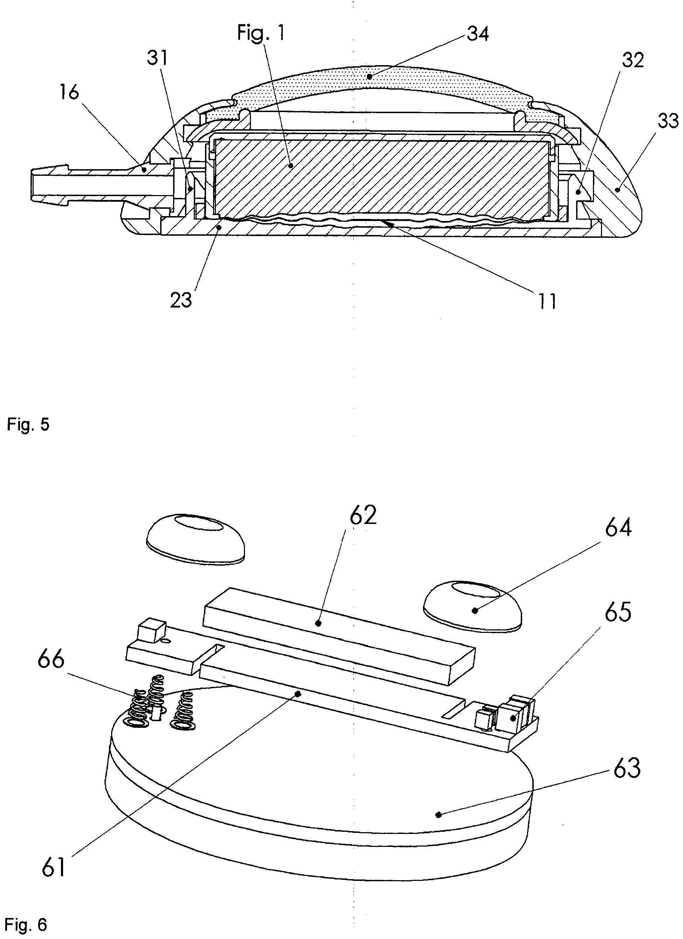

Durch die erfindungsgemäße Montage des Druckaufnehmer Chips und der Trägerplatine ist eine vollständige spannungsfreie Aufhängung gewährleistet. Eine bevorzugte Ausführungsform der Platine ist in Fig. 5 gezeigt.

Um einen weiteren Schutz auch gegen Erschütterungen zu gewährleisten und das Kammervolumen der Messzelle zu minimieren, wird die Messzelle zusätzlich aufgefüllt oder vorzugsweise vergossen. Der Druckaufnehmer selber wird dabei nicht vergossen. Der hierdurch bestehende, direkte Kontakt mit dem druckübertragenden Medium ermöglicht eine hohe Dynamik der Messung, da keinerlei störende Dämpfung zwischen Medium und Sensorchip besteht. Der Spalt zwischen dem Chip und der Füllmaske kann dabei sehr klein gehalten werden, vorzugsweise ist er kleiner als 0,01 mm.

Um Ausgasungen des Füllmaterials nach Verschluss der Messzelle auszuschließen und das Material kontrolliert auszuhärten, wird die Zelle vor Montage der Membran ausgehärtet. Hierzu wird die Zelle mehrere Stunden bei 60 bis 150 Grad Celsius erhitzt. Nach Montage der Membran und damit dem irreversiblen Verschluss der Messzelle ist die Alterung des Füllmaterials nicht mehr zu beeinflussen. Dies ist unkritisch, da die Füllung ausschließlich zum Schutz der empfindlichen Komponenten und der Minimierung des gasgefüllten Kammervolumens dient.

Die erfindungsgemäß gewellte, metallische Membran wird vorzugsweise im kalten Zustand tief gezogen. Dabei wird das Material über die elastische Streckgrenze hinaus verformt, so dass eine bleibende Verformung entsteht. Die Verformung kann in einer Presse zwischen entsprechend geformter Matrize und Patrize erfolgen. Die Verformung kann auch mit einem flüssigen oder gasförmigen Druckmittel erfolgen, mit dem die Folie gegen oder in eine Form gedrückt wird. Die dazu notwendige Matrizenoberfläche bzw. Patrizenoberfläche oder Oberfläche der Form kann im Wege weniger Versuche erstellt werden, bei denen die für die Membranfolie formgebenden Ausnehmungen in der Matrize bzw. Patrize bzw. Formoberfläche so lange vertieft werden, bis die Membran nach der Verformung die gewünschten Wellen zeigt.

Bei Kunststoff ist die bleibende Verformung im kalten Zustand schwierig, weil Kunststoff im kalten Zustand in Abhängigkeit von seiner Beschaffenheit eine extrem große elastische Verformung zeigt. Kunststoffmembranen aus thermoplastischem Material können aber durch Erwärmung zum Erweichen gebracht werden. Im erweichten Zustand ist eine bleibende Verformung leicht.

Wahlweise kann die zusätzliche Membranverformung vor der Wellenformgebung, gleichzeitig mit der Wellenformgebung oder nach der Wellenformgebung erfolgen.

Zum Einbau der Membran wird die Membran an einem Fenster im Gehäuse der Messzelle positioniert und anschließend vorzugsweise mit diesem Gehäuse verschweißt. Das ist sowohl bei Kunststoffteilen wie auch bei metallischen Teilen, auch bei Gehäuse und Membran aus Titan, möglich. Dabei ist günstig als Schweißstelle eine Mantelfläche des Gehäuses zu wählen. An der Mantelfläche kann die Membran mit Ringen oder Hülsen in der Schweißstellung gehalten werden. Die Ringe und Hülsen können bloße Montagehilfen sein und nach dem Schweißen entfernt werden oder bleibend vorgesehen sein.

Bei der Verwendung von Flüssigkeit zur Einbettung der Messeinrichtung hat die metallische Membran bereits Vorteile. Darüber hinausgehende Vorteile der metallischen Membran ergeben sich bei gasförmigen Medien als Druckmedien/Druckmittler. Metallische Membranfolien, insbesondere aus Titan, sind gasdicht. Das ist bei Kunststoffmembranen nicht der Fall. Dort ist mit Diffusion des Gases durch die Kunststoffmembran zu rechnen.

Entsprechend des dargestellten Ausführungsbeispiels ist die Messzelle nach Verschweißen der Membran komplett geschlossen sowie hermetisch dicht verkapselt. Keine elektronischen Bauteile, Zuleitungen, Füllstoffe oder Dichtungen der Zelle haben Kontakt mit dem zu messenden Medium und können hierdurch die Messung beeinflussen.

Die Messzelle wird vorzugsweise als geschlossene Einheit in ein Gehäuse eingebaut. Die Messzelle kann an der Spitze eines Katheters angebracht sein, vorzugsweise handelt es sich um ein extrakraniell zu implantierendes Ventilgehäuse. Eine bevorzugte Bauform zeigt eine Messzelle mit einem Bohrlochreservoir kombiniert. Das Reservoirgehäuse besteht aus einer proximal gelegenen Zuleitung, einer distalen Ableitung, einem Innenraum, der die Messzelle sowie einen Reservoirraum enthält. Das Gehäuse ist abgedichtet und besteht rundum bis auf die Oberseite aus einem festen, dichten und biokompatiblen Material. In Frage hierfür kommt ein Metall, vorzugsweise Titan, besonders bevorzugt ein geeignetes, nichtmetallisches Material, noch weiter bevorzugt ein Polyaryletherketon, am bevorzugtesten Polyetheretherketon (PEEK). Ein nichtmetallisches Gehäuse beeinflusst die induktive Energieversorgung und insbesondere die telemetrische Datenübertragung am wenigsten. Die mögliche Entfernung des (hier nicht beschriebenen) Auslesegerätes vom Implantat erhöht sich hierdurch zum Beispiel auf bis zu zehn Zentimeter. Da das Gehäuse ansonsten keinen Einfluss auf die Funktion der Messzelle hat und auch ansonsten keinen besonderen mechanischen Ansprüchen genügen muss, ist ein nichtmetallisches Material einsetzbar.

Die Oberseite des Gehäuses besteht aus einer Haube aus einem polymeren Material, bevorzugt Silikon.

Die erfindungsgemäße Kombination des Drucksensors mit einem Bohrlochreservoir bietet für den Patienten einen zusätzlichen Vorteil. Der Drucksensor kann vollständig in ein ohnehin vorhandenes Shunt System integriert werden, ein zusätzliches Implantat ist nicht erforderlich. Dadurch wird zugleich der tatsächlich für die Diagnose relevante Druck gemessen, der auch an dem der Ableitungsrichtung folgend implantierten Hydrocephalus Ventil anliegt. Häufig werden in Shunt Systemen Bohrlochreservoire eingesetzt, die die erforderliche Umlenkung des Katheters beim Austritt aus der Schädeldecke übernehmen und über eine bauähnliche Haube aus Silikon verfügen. Derartige Reservoire finden sich beispielsweise im Produktkatalog der Firma Christoph Miethke GmbH&Co. KG. Dadurch ist es möglich, mittels einer Spritze von außen direkt Liquor zu entnehmen oder Medikamente einzubringen. Hierzu kann die Haube direkt durch die Kopfhaut durchstochen werden

Die Messzelle kann derart in das Gehäuse integriert werden, das die flexible Membran zur Unterseite des Gehäuses weist Nach oben und damit zur Haube hin weist die massive Unterseite der Messzelle. Damit kann die Unterseite gleichzeitig als Schutz und Begrenzung beim Durchstoßen der Haube mit einer Spritze dienen. Auch andere Kombination kommen in Betracht, z.B. eine Kombination der Druckmeßzelle mit einem Hydrocephalusventil.

Die gewellte Membran findet auf der Unterseite des Gehäuses eine entsprechend der Wellenform geformte Gegenseite. Bei einem Unterdruck verformt sich die Membran bis sie an der Unterseite anliegt, woraus sich ein Messbereich in etwa gleicher Höhe wie bei einem äußeren Überdruck ergibt. Negative Drücke dieser Größenordnung kommen zwar im Betrieb keinesfalls vor, sie können aber je nach Fertigungsprozess im Fertigungsprozess auftreten.

Die Erfindung eignet sich vorteilhafterweise für Druckbereiche, bei denen es im Wesentlichen zu einer spannungsfreien oder spannungsarmen Verschiebung der Membran kommt.

Grundsätzlich sind verschiedene Messorte zur Hirndruckmessung etabliert. Die intraventriculäre Messung ist möglich, für die parenchymatöse, die epidurale oder die subdurale Messung sind Ausführungsbeispiele ebenfalls nahe liegend.

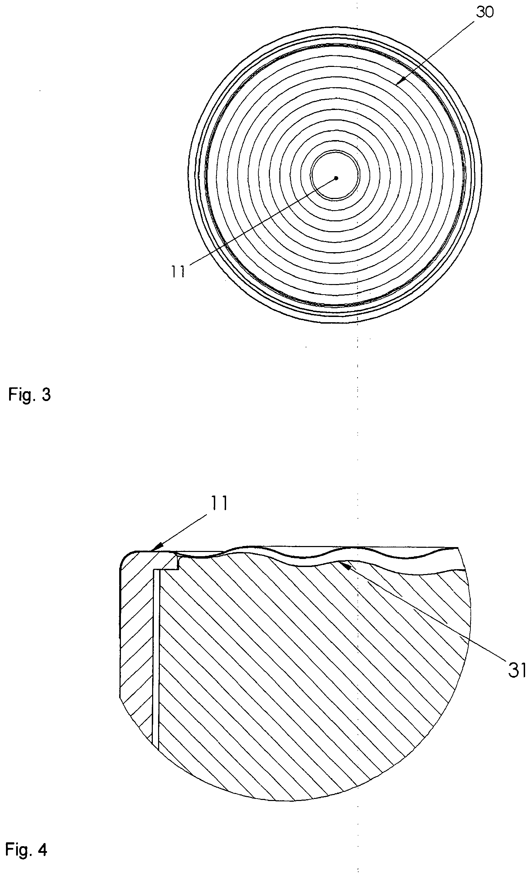

In der Zeichnung ist ein Ausführungsbeispiel der Erfindung dargestellt:

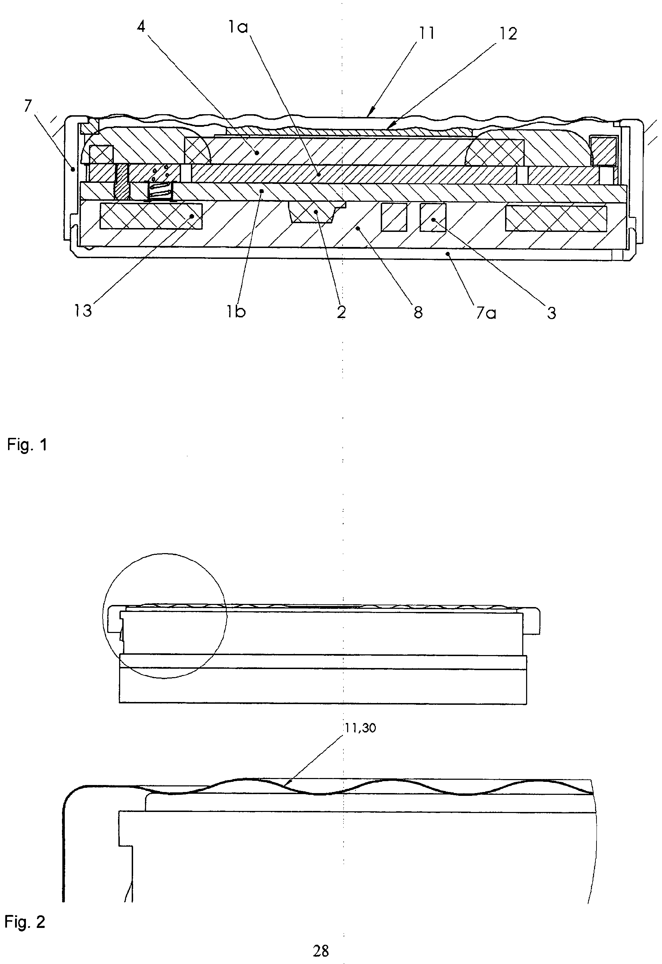

Fig. 1 zeigt den Aufbau eines Drucksensors mit einem Drehteil 7 aus Titan als Gehäuse. Ein Microchip mit Drucksensorik 4, zwei voneinander getrennte Platinen 1a und 1b und weitere elektronische Bauteile 2, 3. Alle Teile sind mit einer Vergussmasse 8 im Gehäuse 7 positionsgesichert.
   Die Folie 11 bildet eine Membran. Unter der Membran besteht ein luftgefüllter Hohlraum, der eine direkte Verbindung mit dem Druckaufnehmer besitzt.
   Die Qualitätssicherung erfolgt mit Hilfe eines Helium-Lecksuchgerätes. Endständig wird das Gehäuse 7 mit einer Kappe 7a verschlossen und verschweißt. Die Elektronikbauteile werden auf der Platine 1 platziert, durch eine Sensor- Spule 13 wird das Senden des Messsignals an eine außen aufgestellte Empfängereinrichtung ermöglicht.
   Die Messvorrichtung misst an der Membran entstehende Hirnflüssigkeitsdruckänderungen, die über die Luftsäule in dem Hohlraum 12 an den Druckaufnehmer weitergegeben werden. Dadurch werden elektrische Signale erzeugt, welche telemetrisch an einen außen liegenden Empfänger weitergegeben werden.
Fig. 2 zeigt eine vergrößerte Einzelansicht des Bereiches mit der Membran 11.
   Im Unterschied zu bekannten Membranen ist die Membran 11 mit Wellen 30 versehen. Die Wellen 30 sind in schematischer Darstellung nach Fig. 3 ringförmig ausgebildet, besitzen einen unterschiedlichen Mittendurchmesser, so dass eine konzentrische Anordnung der Wellen möglich ist. Dabei schließt sich eine Welle an die andere an. Die Wellen 30 verlaufen im Ausführungsbeispiel sinusartig. Bei weiterem Vergleich der Wellen mit sinusartigem Verlauf beträgt die Amplitude im Ausführungsbeispiel 0,8 mm. Diese 0,8 mm verteilen sich zu 0,4 mm auf die Auswölbung der Wellen nach oben und zu 0,4mm auf die Auswölbung der Wellen nach unten. Die oberen Auswölbungen sind aus der Sicht von unten Einwölbungen. Aufgrund der Wellen 30 ist die Membran 11 sehr viel nachgiebiger als eine flache Membran ohne Wellen.

In einem anderen Ausführungsbeispiel nach Fig. 4 ist die Membrananlagefläche 31 der Messeinrichtung dem sich durch Verformung der Membran ergebenden Profil angepasst, so dass sich die nach unten gerichteten Auswölbungen der Wellen 30 in die Vertiefungen in der Membranlagefläche 31 legen und die Erhebungen der Membrananlagefläche 31 sich in die Einwölbungen der Wellen 30 legen. Dadurch wird das nach einer Membranverformung zwischen der Membran 11 und der Membrananlagefläche 31 bestehende Volumen wesentlich verringert.

Die erfindungsgemäße Messeinrichtung eignet sich unter anderem auch für die Erfassung des Flüssigkeitsdruckes in einer Drainageleitung für Liquor in einem Shuntsystem zur Behandlung des Hydrocephalus. Fig. 5 zeigt in einer bevorzugten Ausführungsform eine Übersichtsdarstellung einer solchen Messeinrichtung an einer Dränleitung 16.

Dabei gehören zu der Messeinrichtung
Ein Reservoirgehäuse
Eine Messzelle wie in Fig. 1 beschrieben mit Titanmembran 11

Die vorstehenden Teile wirken wie bei der Messeinrichtung gemäß Fig. 1 bis 3 zusammen. Das Ausführungsbeispiel nach Fig. 5 unterscheidet sich durch das zusätzliche Außen-Gehäuse, welches einen Schutz der Titanmembran 11 durch negative Drücke bildet und eine Druckmessung im geschlossenen Shuntsystem ermöglicht.

Das Reservoirgehäuse in der bevorzugten Ausführungsform nach Fig. 5 besteht im Wesentlichen aus einem rotatorischen Hauptkörper 33, dem Boden 23 mit der gewellten Gegenseite zur Membran 11 und der die Haube 34. Der Auslass erfolgt über die Tülle 16.

Der Boden 23 und verfügt über eine Anzahl Rasthaken 31/32, für die im Hauptkörper sowie im Gehäuse der Messzelle jeweils entsprechende Nuten vorgesehen sind. Vorzugsweise verfügt der Boden über je drei Haken, die am Umfang in einer 120 Grad Teilung versetzt angebracht sind. Andere Einteilungen oder weitere Rasthaken sind möglich. Die Rasthaken 31 für die Montage der Messzelle liegen nach innen hin, die Rasthaken 32 für den Hauptkörper zeigen nach außen. Zur Montage wird die Messzelle in den Boden 23 derart eingelegt, dass die inneren Rasthaken 31 des Bodens in die Nuten der Messzelle einrasten. Dann wird der Boden 23 mit der Messzelle in den Hauptkörper 33 gedrückt bis die Rasthaken in den dafür vorgesehenen Nuten des Hauptkörpers einrasten. Die Haube ist 34 wird dadurch in Ihrer Position fixiert. Diese formschlüssige Positionierung ist durch eine entsprechende Konstruktion der Haken und Nuten spielfrei. Die Montage des Gehäuses erfolgt irreversibel, einmal montiert ist das gesamte Modul nicht mehr zerstörungsfrei zu demontieren. Damit wird die Produktsicherheit signifikant erhöht, da ein unsachgemäßer Umgang mit dem abgenommenen Endprodukt ausgeschlossen ist.

Fig. 6 zeigt in Explosionsansicht den Aufbau der Trägerplatine 61 des ASIC Sensorchips 62 sowie die Montage der Platine auf der Hauptplatine 63. Die Trägerplatine 61 ist geschlitzt zur Abfederung jedweden mechanischen Einflusses auf den Chip 62. Dieser ist nur an einer Stelle mittig mit der Platine 61 verklebt. Die Anschlüsse sind gebondet (nicht dargestellt), die Verbindungen werden durch je einen Glop Top 64 geschützt. Auf der Trägerplatine 61 sind verschiedene Kondensatoren 65 zur Spannungsregelung des ASIC Chips 62 angebracht. Die Trägerplatine 61 ist über spiralfederartig geformte Kontaktdrähte 66 mit der Hauptplatine 63 verbunden.

## Patentansprüche

1. Implantierbare Vorrichtung zur Erfassung von intracraniellen Drücken, wobei ein Druckaufnehmer (4) verwendet wird, der in Wirkverbindung mit einer Messwertübertragung (13) steht, und wobei der Druckaufnehmer ein Mikrochip ist und der Mikrochip sich in einem starren Gehäuse (7) mit einer Druckkammer (12), die ein Kammervolumen aufweist, befindet, wobei zur Druckübertragung ein Fenster in dem Gehäuse vorgesehen ist und das Fenster mit einer dünnen Membran (11) verschlossen ist, wobei die Membran aus einem biokompatiblen Metall besteht,
und wobei die Membran auf ein Gasvolumen als Druckmittler wirkt, das Druckänderungen an der Membran an den Druckaufnehmer weiterleitet, **dadurch gekennzeichnet,**
**dass** die Membran mindestens eine Welle (30) aufweist, wobei die Membrandicke 0,005 bis 0,04mm ist und dass das Kammervolumen, bestehend aus aktivem und passiven Volumen, mindestens 20 Kubikmillimeter und höchstens 350 Kubikmillimeter, vorzugsweise höchstens 130 Kubikmillimeter beträgt.

2. Vorrichtung nach Anspruch 1, **dadurch gekennzeichnet, dass** die Wellen mehrere Auswölbungen bilden, die nach oben und/oder unten gerichtet sind.

3. Vorrichtung nach Anspruch 1 oder 2, **dadurch gekennzeichnet, dass** die Membran zumindest zu 1/3 der druckbeaufschlagten Fläche, vorzugsweise zu 2/3 der druckbeaufschlagten Fläche und höchst bevorzugt zu 4/5 der druckbeaufschlagten Fläche gewellt ist.

4. Vorrichtung nach einem der Ansprüche 1 bis 3, **dadurch gekennzeichnet, dass** die Welle ringförmig verläuft.

5. Vorrichtung nach Anspruch 4, **dadurch gekennzeichnet, dass** mehrere Wellen unterschiedlichen Durchmessers konzentrisch angeordnet sind.

6. Vorrichtung nach Anspruch 4, **dadurch gekennzeichnet, dass** die konzentrisch angeordneten Wellen mindestens teilweise ineinander übergehen.

7. Vorrichtung nach einem der Ansprüche 1 bis 6, **dadurch gekennzeichnet, dass** der Membran eine Membrananlagefläche (31) gegenüberliegt.

8. Vorrichtung nach Anspruch 7, **dadurch gekennzeichnet, daß** die Membrananlagefläche
a)mindestens teilweise eben ist und/oder
b)mindestens teilweise gewölbt und/oder
c)mindestens teilweise trichterförmig und/oder
d)mindestens teilweise hügelförmig ausgebildet ist.

9. Vorrichtung nach Anspruch 8, **dadurch gekennzeichnet, daß** die Membrananlagefläche
gleichfalls gewellt ist, so dass sich die Membran mit ihren gegen die Membrananlagefläche gerichteten Auswölbungen in deren Ausnehmungen legen kann und die Membrananlagefläche sich ihrerseits mit den gegen die Membran gerichteten Auswölbungen in deren Einwärtswölbungen legen kann.

10. Vorrichtung nach einem der Ansprüche 1 bis 9, **dadurch gekennzeichnet, daß** bei Verwendung eines Gasvolumens als Druckmittler zwischen Membran und Druckaufnehmer die Membran einen aktiven Hubraum aufweist, welcher größer als das Gasvolumen ist, welches nach maximaler Verformung der Membran zwischen der Membran und dem Druckaufnehmer besteht.

11. Vorrichtung nach Anspruch 10, **dadurch gekennzeichnet, daß** das aktive Hubvolumen höchsten 4fach größer, noch weiter bevorzugt höchsten 2fach größer als der passive Teil des Kammervolumens ist, wobei der passive Teil des Kammervolument das Gasvolumen ist, welches nach maximaler Verformung der Membran zwischen der Membran und dem Druckaufnehmer besteht.

12. Vorrichtung nach einem der Ansprüche 1 bis 11, **dadurch gekennzeichnet, daß** bei kreisförmiger Membran der maximale Radius der Membran mindestens 15fach, vorzugsweise höchstens 50fach größer als der maximale Membranhub ist.

13. Vorrichtung nach einem der Ansprüche 1 bis 5, **dadurch gekennzeichnet, dass** die Wellen Auswölbungen besitzen, die nach oben und/oder unten gerichtet sind und an den Auswölbungen eine Rundung besitzen, deren Radius mindestens gleich der mehrfachen Dicke der Membran ist, vorzugsweise gleich der 10fachen Dicke der Membran ist und noch weiter bevorzugt mindestens gleich der 100fachen Dicke der Membran ist.

14. Vorrichtung nach einem der Ansprüche 7 bis 13, **dadurch gekennzeichnet, daß** die Membran in einem Druckbereich von 800 bis 1200 mbar ohne Berührung mit der Membrananlagefläche ist.

15. Verfahren zur Herstellung der Vorrichtung nach einem der Ansprüche 1 bis 14, dass bei Verwendung einer metallischen Membran die Membran zur Wellung kalt bis über die elastische Streckgrenze tiefgezogen wird.

16. Verfahren nach Anspruch 15 **dadurch gekennzeichnet, dass** die Verformung der Membran zur Wellenbildung an einer Formfläche erfolgt, die der gewünschten Wellenbildung nachgeformt ist, wobei die Konturen der Formfläche so lange vertieft werden, bis sich die gewünschte Wellenbildung an der Membran einstellt.

17. Verfahren nach Anspruch 15 oder 16, **dadurch gekennzeichnet, dass** die Membrananlagefläche der verformten Membran nachgebildet wird.

## Claims

1. Implantable device for detecting intracranial pressures, wherein a pressure sensor (4) is used which is in operative connection with a measurement value transmission (13), and wherein the pressure sensor is a microchip and the microchip is located in a rigid housing (7) with a pressure chamber (12) which has a chamber volume, wherein a window is provided in the housing for pressure transmission and the window is closed with a thin membrane (11), wherein the membrane is made of a biocompatible metal, and wherein the membrane acts as a diaphragm seal on a gas volume which transmits pressure changes on the membrane to the pressure sensor, **characterized in that** the membrane has at least one shaft (30), wherein the membrane thickness is 0.005 to 0.04 mm and that the chamber volume, consisting of active and passive volume, is at least 20 cubic millimeters and at most 350 cubic millimeters, preferably at most 130 cubic millimeters.

2. Device according to claim 1, **characterized in that** the shafts form several bulges which are directed upwards and/or downwards.

3. Device according to claim 1 or 2, **characterized in that** the membrane is corrugated at least to 1/3 of the pressurized area, preferably to 2/3 of the pressurized area and most preferably to 4/5 of the pressurized area.

4. Device according to one of claims 1 to 3, **characterized in that** the shaft is annular.

5. Device according to claim 4, **characterized in that** several shafts of different diameters are arranged concentrically.

6. Device according to claim 4, **characterized in that** the concentrically arranged shafts at least partially merge into each other.

7. Device according to any one of claims 1 to 6, **characterized in that** a membrane contact surface (31) lies opposite the membrane.

8. Device according to claim 7, **characterized in that** the membrane contact surface
a) is at least partially flat and/or
b) is at least partially curved and/or
c) is at least partially funnel-shaped and/or
d)is at least partially hill-shaped.

9. Device according to claim 8, **characterized in that** the membrane contact surface is also corrugated, so that the membrane can lie with its bulges directed against the membrane contact surface in the recesses of the membrane contact surface and the membrane contact surface can in turn lie with the bulges directed against the membrane in the inward bulges of the membrane.

10. Device according to one of claims 1 to 9, **characterized in that** when using a gas volume as a diaphragm seal between the membrane and the pressure sensor, the membrane has an active displacement which is greater than the gas volume which exists between the membrane and the pressure sensor after maximum deformation of the membrane.

11. Device according to 10, **characterized in that** the active displacement volume is at most 4 times larger, still preferably at most 2 times larger than the passive part of the chamber volume, the passive part of the chamber volume being the gas volume which exists between the membrane and the pressure sensor after maximum deformation of the membrane.

12. Device according to one of claims 1 to 11, **characterized in that** in the case of a circular membrane the maximum radius of the membrane is at least 15 times, preferably at most 50 times, greater than the maximum membrane stroke.

13. Device according to one of claims 1 to 5, **characterized in that** the shafts have bulges which are directed upwards and/or downwards and have a rounding at the bulges, the radius of which is at least equal to several times the thickness of the membrane, preferably equal to 10 times the thickness of the membrane and still further preferably at least equal to 100 times the thickness of the membrane.

14. Device according to one of claims 7 to 13, **characterized in that** the membrane is without contact with the membrane contact surface in a pressure range from 800 to 1200 mbar.

15. Method for producing the device according to one of claims 1 to 14, in that, when using a metallic membrane, the membrane is deep-drawn cold to above the elastic yield point for corrugation.

16. Method according to claim 15, **characterized in that** the deformation of the membrane for corrugation takes place on a forming surface which is shaped to resemble the desired corrugation, the contours of the forming surface being deepened until the desired corrugation is produced on the membrane.

17. Method according to claim 15 or 16, **characterized in that** the membrane contact surface of the deformed membrane is reproduced.

## Revendications

1. Dispositif implantable pour la détection de pressions intracranuelles, dans lequel on utilise un capteur de pression (4) qui est en liaison active avec une transmission de valeurs de mesure (13), et dans lequel le capteur de pression est une micropuce et la micropuce est située dans un boîtier rigide (7) avec une chambre de pression (12) qui présente un volume de chambre, dans lequel une fenêtre est prévue dans le boîtier pour la transmission de pression et la fenêtre est fermée par une fine membrane (11), la membrane étant en un métal biocompatible, et la membrane agissant comme médiateur de pression sur un volume de gaz qui transmet au capteur de pression les variations de pression sur la membrane, **caractérisé en ce que** la membrane présente au moins une onde (30), l'épaisseur de la membrane étant de 0,005 à 0,04 mm et **en ce que** le volume de la chambre, constitué d'un volume actif et d'un volume passif, est d'au moins 20 millimètres cubes et d'au plus 350 millimètres cubes, de préférence d'au plus 130 millimètres cubes.

2. Dispositif selon la revendication 1, **caractérisé en ce que** les ondes forment plusieurs renflements qui sont dirigés vers le haut et/ou vers le bas.

3. Dispositif selon la revendication 1 ou 2, **caractérisé en ce que** la membrane est ondulée au moins à 1/3 de la surface pressurisée, de préférence à 2/3 de la surface pressurisée et de préférence à 4/5 de la surface pressurisée.

4. Dispositif selon l'une des revendications 1 à 3, **caractérisé en ce que** l'onde est annulaire.

5. Dispositif selon la revendication 4, **caractérisé en ce que** plusieurs ondes de diamètres différents sont disposés concentriquement.

6. Dispositif selon la revendication 4, **caractérisé en ce que** les ondes disposées concentriquement se confondent au moins partiellement.

7. Dispositif selon l'une des revendications 1 à 6, **caractérisé en ce qu'**une surface de contact (31) de la membrane se trouve en face de la membrane.

8. Dispositif selon la revendication 7, **caractérisé en ce que** la surface de contact de la membrane
a) est au moins partiellement plane et/ou
b) est au moins partiellement courbée et/ou
c) est au moins partiellement en forme d'entonnoir et/ou
d) est au moins partiellement en forme de colline.

9. Dispositif selon la revendication 8, **caractérisé en ce que** la surface de contact de la membrane est également ondulée de sorte que la membrane peut reposer dans les creux de la surface de contact de la membrane avec ses renflements dirigés vers la surface de contact de la membrane et la surface de contact de la membrane peut à son tour reposer dans les renflements intérieurs de la membrane avec ses renflements dirigés vers la membrane.

10. Dispositif selon l'une des revendications 1 à 9, **caractérisé en ce que**, lors de l'utilisation d'un volume de gaz comme médiateur de pression entre la membrane et le capteur de pression, la membrane a un déplacement actif qui est plus grand que le volume de gaz qui existe entre la membrane et le capteur de pression après déformation maximale de la membrane.

11. Dispositif selon 10, **caractérisé en ce que** le volume de déplacement actif est au maximum 4 fois plus grand, et même de préférence au maximum 2 fois plus grand que la partie passive du volume de la chambre, la partie passive du volume de la chambre étant le volume de gaz qui existe entre la membrane et le capteur de pression après déformation maximale de la membrane.

12. Dispositif selon l'une des revendications 1 à 11, **caractérisé en ce que**, dans le cas d'une membrane circulaire, le rayon maximal de la membrane est au moins 15 fois, de préférence au maximum 50 fois, supérieur à la course maximale de la membrane.

13. Dispositif selon l'une des revendications 1 à 5, **caractérisé en ce que** les ondes présentent des renflements qui sont dirigés vers le haut et/ou vers le bas et présentent un arrondi au niveau des renflements, dont le rayon est au moins égal à plusieurs fois l'épaisseur de la membrane, de préférence égal à 10 fois l'épaisseur de la membrane et encore de préférence au moins égal à 100 fois l'épaisseur de la membrane.

14. Dispositif selon l'une des revendications 7 à 13, **caractérisé en ce que** la membrane se trouve dans une plage de pression de 800 à 1200 mbar sans contact avec la surface de contact de la membrane.

15. Procédé de fabrication du dispositif selon l'une des revendications 1 à 14, en ce que, lors de l'utilisation d'une membrane métallique, la membrane est emboutie à froid jusqu'à dépasser la limite élastique d'ondulation.

16. Procédé selon la revendication 15, **caractérisé en ce que** la déformation de la membrane pour l'ondulation a lieu sur une surface de formage qui est façonnée pour ressembler à l'ondulation souhaitée, les contours de la surface de formage étant approfondis jusqu'à ce que l'ondulation souhaitée soit produite sur la membrane.

17. Procédé selon la revendication 15 ou 16, **caractérisée en ce que** la surface de contact de la membrane est modelée sur la membrane déformée.
